# EUROPEAN PATENT SPECIFICATION

(11) **EP 1 228 077 B1**
(45) Date of publication and mention of the grant of the patent: **11.06.2003**
(21) Application number: 00975930.9
(22) Date of filing: 26.10.2000
(51) Int. Cl.: C07F 15/00

(54) **RUTHENIUM (II) COMPLEXES WITH HIGH ANTITUMORAL AND ANTIMETASTATIC ACTIVITIES**
RUTHENIUM (II) KOMPLEXE MIT HOHER ANTITUMOR UND ANTIMETASTATISCHER WIRKUNG
COMPLEXES DE RUTHENIUM (II) PRESENTANT DES ACTIVITES ANTITUMORALE ET ANTIMETASTATIQUE ELEVEES

(30) Priority: 28.10.1999 IT MI992256
(43) Date of publication of application: 07.08.2002
(73) Proprietor: Sigea S.R.L., 34012 Trieste (IT)
(72) Inventor: MESTRONI, Giovanni, I-34127 Trieste (IT); ALESSIO, Enzo, I-34131 Trieste (IT); SAVA, Gianni, I-34143 Trieste (IT); IENGO, Elisabetta, I-34143 Trieste (IT); ZORZET, Sonia, I-34075 San Canzian d'Isonzo-Pieris (IT); BERGAMO, Alberta, I-34128 Trieste (IT)
(74) Representative: Gervasi, Gemma, Dr.
(86) International application number: EP0010566
(87) International publication number: WO01030789

(56) References cited:
- WO-A-98/00431
- IENGO, ELISABETTA ET AL: "Novel ruthenium(III) dimers Na2[{trans-RuCl4(Me2SO-S)}2(.mu.-L)] and [{mer,cis-RuCl3(Me2SO-S)(Me2SO-O)}2(.mu.-L )] (L = bridging heterocyclic N-donor ligand) closely related to the antimetastatic complex Na[trans-RuCl4(Me2SO-S )(Him)]" J. CHEM. SOC., DALTON TRANS. (1999), (19), 3361-3371 , XP000917158
- ALESSIO, E. ET AL.: "Synthesis ans characterization of two new classes of ruthenium(III)-sulfoxide complexes with nitrogen donor ligands (L)...."" INORGANICA CHIMICA ACTA, no. 203, 1993, pages 205-205-217, XP002041111

## Description

### Field of the invention

The present invention relates to the use in therapy of a class of Ruthenium (II) complexes, specifically for the treatment of tumours characterised by high metastasizing ability.

### Background Art

The search for new antitumoural compounds is in continuous development and is mainly aimed at the identification of new compounds with high selectivity, with high antimetastatic activity and with reduced toxicity for the host.

Compounds based on transition metals, such as cisplatin for example, have been used for many years in the chemotherapy of tumours; these products, although pharmacologically active, have a reduced antimetastatic activity and show heavy side-effects caused by the elevated systemic toxicity.

Recently, the applicative potentialities of some ruthenium complexes (II) with cytotoxic activity and their possible use in the therapy of neoplastic diseases has been considered. Unfortunately, even if these compounds show a higher tropism for the tumours than the cisplatin derivatives (Sava et al, Anticancer Res. 11, 1103, 1991), they show also toxic effects for the organism.

In order to improve the problems related to the activity and toxicity, new Ruthenium (III) complexes have been proposed in the form of more active and less toxic pro-drugs (Sava et al., Anticancer Res. 11, 1103, 1991, Sava et al. in Topics in Biological Inorganic Chemistry, 143, 1999) for which an "in vivo" activation mechanism by reduction of the Ruthenium (III) complexes to the corresponding Ruthenium (II) reactive species has been devised.

This mechanism has been hypothesized to be more efficient in the hypoxic and reducing environment of the tumour tissue, and this would allow for the high selectivity and activity of the Ruthenium (III) complexes towards solid tumours (Sava et al., Anticancer Res. 11, 1103, 1991, Sava et al. in Topics in Biological Inorganic Chemistry, 143, 1999).

The reduction process would occur to a lesser extent in healthy tissues which are normally vascularized and where the partial pressure of oxygen is higher (40 mm Hg) than in the tumour tissue (5 mm Hg). Higher amounts of the more active and toxic species of Ruthenium (II) are formed in the tumour tissue with respect to the healthy tissues, with an accumulation effect resulting in a selective cytotoxicity towards the solid tumour cells.

Among the above mentioned Ruthenium (III) complexes, the ImH[trans-RuCl₄Im₂], (B.K. Keppler et al., J. Cancer Res. Clin. Oncol., 111: 166-168, 1986), the complexes of formula Na[trans-RuCl₄(Me₂SO)(L)], (WO 90/13553) proved to be effective in slowing down the growth of the primary tumour and the most recent complexes of formula (LH) [trans-RuCl₄(Me₂SO)(L)] (WO98/00431) that show a marked antimetastatic activity.

However, despite the positive results obtained in clinical applications in chemotherapy, the search in the field of ruthenium derivatives is still open to the identification of new compounds with optimal features of cytotoxicity and selectivity toward the tumour and with a reduced systemic toxicity in order to render the use of these compounds safer and more effective.

### Summary of the invention

The present invention refers to the use in therapy of Ruthenium (II) anionic complexes with antitumour and antimetastatic activity having formula I: 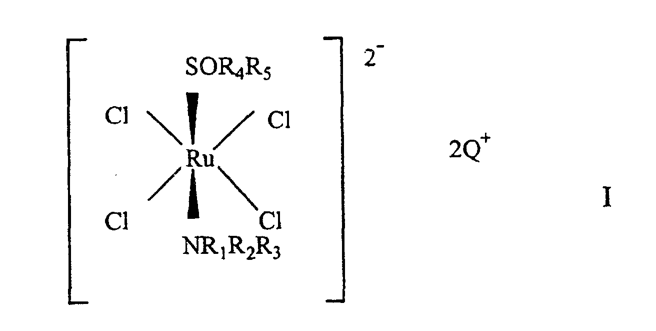 wherein,
**R**_{**1**}, **R**_{**2**}, **R**_{**3**} either the same or different from one another, are selected in the group consisting of: H, C₁-C₆ alkyl, either linear or branched, saturated or unsaturated, C₃-C₇ cycloalkyl, aryl;
or NR₁R₂R₃ is a 5-7-membered nitrogen heterocycle, either saturated or unsaturated, possibly containing one or more O,S, N atoms or N substituted with an alkyl, aryl or benzyl residue; said nitrogen heterocycle can be possibly benzocondensed and/or substituted with C₁-C₄ alkyl, C₁-C₄ alkoxy, C₁-C₄ alkylthio, aryl or benzyl;
the counterion Q⁺ is represented by ⁺NHR₁R₂R₃, wherein R₁ R₂ R₃ maintain the aforementioned meanings;
**R**_{**4**} ed **R**_{**5**}, either the same or different from one another are selected in the group consisting of: H, C₁-C₆ alkyl, C₃-C₇ cycloalkyl , aryl; or R₄ and R₅ form together with the S atom to which they are bound, a 4-7-membered heterocycle.

The Applicant has surprisingly found that the Ruthenium (II) complexes according to the invention, show unexpected antitumour activities in terms of activity and therapeutical index.

A further object of the present invention is represented by pharmaceutical compositions containing the Ruthenium (II) complexes of formula I as the active compound, in association with suitable excipients and/or diluents and/or stabilizers.

In a further embodiment, the invention provides a kit for the preparation of the Ruthenium (II) compounds having formula I.

In a further embodiment the invention provides for the use of the formula (I) Ruthenium (II) complexes to prepare a medicament for the treatment of solid tumors in particular those characterized by high metastatizing properties.

### Detailed description of the invention

Object of the present invention is the use of Ruthenium (II) complexes of formula I for therapeutic purposes: 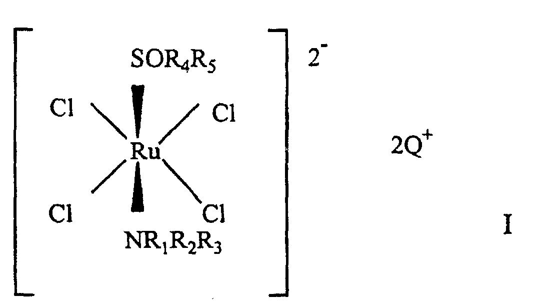 wherein the meanings of R₁, R₂, R₃, R₄, R₅ and Q⁺ are hereinafter reported.

The **R**_{**1**}, **R**_{**2**}, **R**_{**3**} groups, either different or the same, are selected in the group consisting of: H, C₁-C₆ alkyl, either linear or branched, saturated or unsaturated, C₃-C₇ cycloalkyl, aryl;
or NR₁R₂R₃ is a 5-7-membered nitrogen heterocycle, either saturated or unsaturated, possibly containing one or more O, S, N atoms, N-substituted with an alkyl, aryl or benzyl residue; said nitrogen heterocycle can possibly be benzocondensed and/or substituted with C₁-C₄ alkyl, C₁-C₄ alkoxy, C₁-C₄ alkylthio, aryl or benzyl.

When NR₁R₂R₃ is a 5-membered nitrogen heterocycle, it is preferably selected in the group consisting of: imidazole, N-methylimidazole, pyrazole and oxazole; more preferably said nitrogen heterocycle is imidazole.

When NR₁R₂R₃ is a 6-membered heterocycle, it is selected preferably in the group consisting of: pyridine, 3,5-lutidine and 4-methylpyridine.

When NR₁R₂R₃ is a 7-membered heterocycle, it is selected preferably in the group consisting of: azepine, diazepine and oxazepine.

Finally, when said heterocycle is benzocondensed, it is selected preferably in the group consisting of: indazole, isoquinoline, benzimidazole and 1,5,6-trimethyl-benzimidazole.

In the complexes according to the invention, **Q**^{**+**} represents a NH⁺R₁R₂R₃ nitrogen group wherein R₁, R₂ and R₃ maintain the meanings assigned above.

In the complex according to the present invention, the sulfoxide ligand **R**_{**4**}**-SO-R**_{**5**} has R₄ and R₅, either the same or different from one another, selected in the group consisting of: H, C₁-C₆ alkyl, C₃-C₇ cycloalkyl, aryl; or R₄ and R₅ form, together with the S atom to which they are bound, a 4-7 membered heterocycle. In the complexes according to the present invention, the sulfoxide ligand R₄-SO-R₅ is preferably dimethylsulfoxide (R₄=R₅=methyl) or diethylsulfoxide (R₄=R₅=ethyl). These products can be obtained by reduction of the corresponding Ruthenium (III) complexes, wherein with a corresponding Ruthenium (III) complex, the following formula II compound is meant:

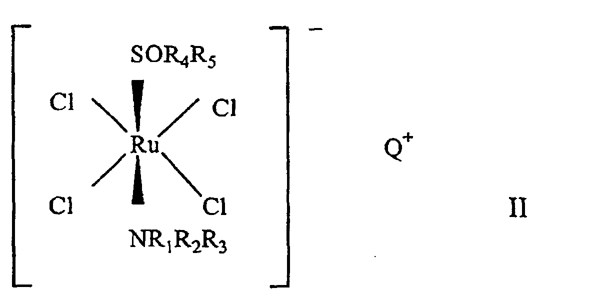

Wherein said R₁, R₂, R₃, R₄, R₅ and Q⁺ groups maintain the above mentioned meanings. Formula II compounds are obtainable for example as described in WO 98/00431 herein incorporated as a reference.

The Ruthenium (II) complexes according to the invention are surprisingly more active than the known Ruthenium (II) complexes and than the corresponding Ruthenium (III) compounds of formula II, and unexpectedly less toxic than both complexes.

In a further embodiment, the present invention provides pharmaceutical compositions containing, as the active component, the Ruthenium (II) complex of formula 1, in combination with suitable excipients, diluents or pharmacologically acceptable stabilizers.

The pharmaceutical compositions are in the form of solution or suspension, or else in the form of gel, grain powder, tablets, pills, capsules or inserts.

The pharmaceutical compositions according to the invention may contain, besides the compounds of formula I, one or more antitumour drugs, such as cisplatin for example, vincristine, vinblastin, 5-fluoruracyl, cyclophosphamide, bleomycin, anthracyclin, taxole or other ruthenium complexes. In a preferred embodiment the pharmaceutical compositions of the invention are prepared at the moment of use or immediately before use by reduction of the corresponding Ruthenium (III) complexes of formula II with reducing agents, selected preferably in the group of physiologically compatible reducing agents.

These reducing agents are selected among those with a redox potential wherein the sum of the two semipotentials (the sum of the reduction potential of the complex and the potential of the reducing agent with opposite sign) is positive so as to ensure an effective reduction. Potential measurements are performed as reported in Alessio et al. Inorganica Chimica Acta, 1993, 203: 205-217.

In a preferred embodiment the physiologically compatible reducing agents are selected in the group consisting of: ascorbic acid, cysteine and glutathione.

Reduction is performed by using a ratio of equivalents between the Ruthenium (III) complex and the reducing agent ranging from 5:1 to 1:2 (the number of equivalents of a substance participating into a redox reaction has been obtained by dividing the grams of said substance for its equivalent weight. In turn, its equivalent weight has been obtained by dividing the molecular weight of the substance for the variation of the oxidation number).

The ratio of equivalents between the Ruthenium (III) complex and the reducing agent is preferably 1. Under these conditions, the Ruthenium (III) reduction to Ruthenium (II) is complete and immediate.

As an alternative, the reduction of the Ruthenium (III) complex of formula II can take place in the presence of inorganic reducing agents such as for example Sn²⁺ or Ce³⁺ or by electrochemistry, or else in the presence of H₂.

In one of its further embodiments, the invention provides a kit for the preparation of the Ruthenium (II) complex of formula I with two components, wherein the first component is the corresponding Ruthenium (III) complex of formula II and the second is a reducing agent, as described above. Both kit components are in the form of solutions present in separate containers to be mixed at the moment of use or immediately before use, or in a solid form as single compounds to be solubilized with suitable solvents when used or immediately before use; as an alternative, the kit is composed of two containers the content of which is to be mixed at the moment of use or immediately before use and wherein the former contains the formula II compound that has been mixed in a dry form with the reducing agent and the latter contains the solvent.

The solvents used for the preparation of both the solution of the Ruthenium (III) complex and the reducing agent are aqueous solvents, such as isotonic solutions of 0.9% NaCI or buffered solutions such as phosphate buffer, citrate buffer etc. and are preferably the same in both solutions. Preferably, the Ruthenium (III) complex of formula II is dissolved in a water solution at a concentration ranging from 0.1 to 20 g/l and with a pH ranging from 3 to 8.

In a further embodiment the invention refers to the use of the formula I Ruthenium (II) complex of for the preparation of medicaments to be used in the prevention and treatment of tumours and metastases. The use of these compounds according to the invention has proved to be very effective on solid tumours, specifically on those characterized by high metastasising ability, especially those selected in the group consisting of: colon carcinomas or tumours of the gastrointestinal tract, mammary carcinomas, lung tumours or lung metastases from highly invasive tumours.

Parenteral, oral, topical or transdermal administration are preferred. Preferably, the Ruthenium (II) complex is prepared at the moment of use or immediately before use, by mixing the corresponding Ruthenium (III) complex with a reducing agent, as previously described.

Preferably, the reducing agent is selected in the group consisting of: ascorbic acid, cysteine, glutathione. The mixing can be carried out by mild mechanical stirring or by another method known to the skilled artisan. For the illustrative but not limitative purposes of the invention, the following examples are reported.

### EXPERIMENTAL PART:

### Preparation of the formula I Ruthenium (II) complexes.

### EXAMPLE 1: Preparation of the Ruthenium (II) complex by reduction of {trans-RuCl₄(Me₂SO)(Im)}ImH (Im=imidazole) with ascorbic acid.

To a pH=7.2 buffered 0.15 M NaCl solution of {trans-RuCl₄(Me₂SO)(Im)}ImH (lm= imidazole) (10g/L) an equivalent amount of ascorbic acid is added. Soon after the mixing, the solution obtained has been analyzed by means of 1H-NMR. The recorded spectrum shows the total reduction of the Ruthenium (III) nucleus to Ruthenium (II) with an extremely fast reaction kinetics.

The fact that the signal corresponding to Me₂SO coordinated with ruthenium is read at 3.60 ppm, (whereas the Me₂SO signal coordinated with the Ruthenium (III) complex must be at -15 ppm) and that its spectral width is equal to 3Hz (while the corresponding value in the spectrum of the Ruthenium (III) complex must be 370 Hz) demonstrated the conversion of the Ruthenium nucleus from paramagnetic to diamagnetic.

Moreover, in the spectrum further coordinated imidazolium signals (8.53 ppm, H2; 7.83 and 7.54 ppm H4,H5) and free imidazolium (8.46 ppm, H2; and 7.39 ppm H4, H5) were observed. The signals at 4.83, 4.67, 4,35 ppm corresponded to the signals of the oxidized ascorbic acid.

According to the above information, it turned out that the reduction of the ruthenium (III) complex has produced the dianionic species: {trans-RuCl₄(Me₂SO)}(Im)}²⁻, corresponding to Ruthenium (II).

### EXAMPLE 2: Preparation of the Ruthenium complex (II) by reduction of the {trans-RuCl₄(Me₂SO)(Pyz)}PyzH (Pyz=pyrazine) with ascorbic acid.

To an aqueous solution of the Ruthenium (III) complex {trans-RuCl₄(Me₂SO)(Pyz)}PyzH}(Pyz=pyrazine) in 0.1 M phosphate buffer pH=7.4, an equivalent amount (1:1) of ascorbic acid has been added. Immediately after mixing, the solution obtained is analyzed by means of 1H-NMR. The spectrum recorded highlighted the total reduction of the Ru(III) to Ru(II) nucleus, accordingly to what observed in the previous example. The multiplet signals at 9.66 and 8.70 ppm of the spectrum highlighted the presence of pyrazine coordinated to the Ruthenium of the {trans-RuCl₄(Me₂SO)(Pyz)}²⁻dianonic species. The signals of the free PyzH⁺ free are read at 8.69 ppm. The protons of Me₂SO coordinated to Ruthenium (II) have been detected at about 3.65 ppm.

### EXAMPLE 3: Preparation of the Ruthenium (II) complex by reduction of the {trans-RuCl₄(Me₂SO)(Im)}ImH with ascorbic acid (equivalent ratio = 2.5:1).

An aqueous solution of the Ruthenium {transRuCl₄(Me₂SO)(Im)}(ImH) (1,832 g/L) containing ascorbic acid (1.41 10⁻² g/L) has been prepared. Immediately after the stirring, the solution was transferred in a quartz cuvette (path length: 1cm) and the UV/VIS spectra was recorded at defined lenghts of time. The decrease in absorbance (Abs) at 390 nm, the main absorption band demonstrated the Ruthenium (III) complex reduction to Ruthenium (II).

### Biological Activity Assays

### EXAMPLE 4. In vivo activity assays of the compositions of the ruthenium (II) complexes according to the present invention on mice affected by MCa mammary carcinoma: prevention activity on the formation of lung metastases.

Six groups composed of 6 CBA\Lac kin female mice, weighing 23±3 g were inoculated on day 0 with 10⁶ viable cells (as determined with Trypan blue-exclusion test) of MCa mammary carcinoma, in 0.05 ml volume of Dulbecco's buffered at pH 7.4 calcium and magnesium-free saline physiological solution (D-PBS), by intramuscular injection with a sterile insulin syringe. The tumour cells derive from donors of the same strain, were transplanted with an insulin sterile syringe accordingly to the same procedure two weeks before. The tumour cells in PBS suspension were prepared by mechanical mincing of the tumour mass obtained from donor mice and by further removing tissue and cell debris by filtration through a double layer of sterile gauze and further centrifugation at 250xg for 10 minutes. The kin animals were derived from a colony obtained from Chester Beatthy, London (UK) and grown in the University of Trieste pound according to the procedures for the rearing of kin animals. The MCa mammary carcinoma tumour line derives from a stock, kept in liquid nitrogen, of tumour cells obtained originally from Rudjer Boskovic Institute, Zagreb (HR).

From day 12 to day 17 after tumour implantation, the six groups of mice were treated intraperitoneally using an insulin sterile syringe, respectively with:
Group 1. Control: 10 mg/kg body weight/die of sterile pyrogen-free physiological solution;
Group 2. ASC: 6.69 mg/kg body weight/die of ascorbic acid in sterile and pyrogen-free physiological solution;
Group 3. CYST: 12 mg/kg body weight/die of cysteine in sterile and pyrogen-free physiological solution;
Group 4. RUT: 35 mg/kg body weight/die of Ruthenium (III) complex, (lmH)[trans-RuCl₄(Me₂SO)(Im)] in pyrogen-free and sterile physiological solution;
Group 5. RUT-ASC: 35 mg/kg body weight/die of the Ruthenium (III) complex, (ImH)[trans-RuCl₄(Me₂SO)(Im)] and 12 mg/kg body weight/die of cysteine in sterile and pyrogen-free physiological saline solution;
Group 6. RUT-CYST: 35 mg/kg body weight/die of the Ruthenium (III) complex, (ImH)[trans-RuCl₄(Me₂SO)(Im)] and 12 mg/kg body weight/die of cysteine in sterile and pyrogen-free physiological saline solution.

The reducing agents ascorbic acid and cysteine were used in a equivalent ratio corresponding to 1 with the Ruthenium (III) complex. The redox reaction was immediate. The administration took place after analytical control of the complete reduction of the Ruthenium (III) complex to Ruthenium (II).

On day 17 from tumour implantation, the growth of the primary tumour was evaluated and the tumor surgically removed after general anaesthesia of the animals with ketamine. The tumor size was determined by measuring both orthogonal axes of the tumour by means of a caliber, the weight in grams of the rotation solid developed around these axes was measured, considering the tumour density equal to 1 and using the formula. (π/6).a².b, wherein a was the minor and b the major axis.

On day 21 from tumour implantation, the mice were sacrificed by cervical dislocation and lung metastases were analyzed. The lungs removed from the animal immediately after sacrifice, were divided in single lobes, which were then examined immediately with a low-magnification microscope equipped with an ocular-grid for the measurement of the metastases sizes from which the two orthogonal a and b axes (with a ≤b) are identified. The metastases were then grouped on the basis of their dimensions and the metastatic tumour weight was calculated as the sum of the weight of each single metastasis, each of them regarded as a rotation solid developed around the above mentioned axes, and with a volume calculated by the same formula used for the primary tumour. The experimental data obtained were therefore processed by appropriate statistical tests. The results are reported in Table 1 wherein the number and weight of lung metastases obtained in each group of animal are shown.

**Table 1**

| Treatment | Metastases | | | |
|---|---|---|---|---|
| | Number | Weight (mg) | %T/C | a.p./a.t |
| 1 Controls | 24.2 ± 7.4 | 92.0±17 | 100 | 0/6 |
| 2 ASC | 18.3± 8.6 | 81.5± 14 | 86 | 0/6 |
| 3 CYST | 35.0± 13.5 | 94.6± 26 | 103 | 0/6 |
| 4 RUT | 7.0± 1.9 | 15.2± 2.3 | 16 | 0/6 |
| 5 RUT + ASC | 4.0 | 15.2 | 16 | 5/6 |
| 6 RUT + CYST | 3.5± 1.0 | 9.2± 2.0 | 10 | 2/6 |
| %T/C = percentage of the metastases weight in the treated animals / metastases weight in the controls; a.p./a.t.: number of animals devoid of metastases/ total number of animals. | | | | |

The data reported in Table 1 show that cysteine (group 3) and ascorbic acid (group 2) when used alone do not produce any relevant effect on lung metastases, while the Ruthenium (III) complex (group 4) and the compositions of the Ruthenium (II) complexes according to the invention (groups 5 and 6) caused a marked reduction in the number and an even more marked decrease in the weight of lung metastases with respect to the controls. It is evident that the compositions of Ruthenium (II) according to the inventions (groups 5 and 6) have a more pronounced effect than the corresponding Ruthenium (III) (group 4) on the number of metastases. With regard tothe metastases weight, the stronger effect was seen in the compositions containing cysteine as a reducing agent (group 6). It is moreover relevant that, with respect to the treatment with the Ruthenium (III) complexes according to the prior art (group 4), the use of Ruthenium (II) complexes according to the invention (groups 5 and 6) prevented the formation of metastases as shown by the ap/at ratio between animals devoid of metastases and number of total animals with respect to the control treatments and as confirmed by statistical analysis (Fisher test) .

### EXAMPLE 5. In vivo activity assay of the compositions according to the present invention on mice affected by MCa mammary carcinoma: prevention activity in the formation of lung metastases.

In a further experiment similar to the previous one, six groups of animals, each of them composed of 7 CBA/Lac kin female mice, weighing 23±3 g were inoculated on day 0 with cells of MCa mammary carcinoma, according to the protocol described in the previous example. From day 10 to day 15 after tumour implantation, the 6 groups of mice were treated intraperitoneally using a sterile insulin syringe, respectively with:
Group 1: Control: 10 ml/kg body weight/die of sterile and pyrogen-free physiological solution;
Group 2: GLU: 23.5 mg/kg body weight/die of glutathione in sterile and pyrogen-free physiological solution;
Group 3: CYST: 12 mg/kg body weight/die of cysteine in sterile and pyrogen-free saline physiological solution;
Group 4: RUT: 35 mg/kg body weight/die of Ruthenium (III) complex, (ImH)[trans-RuCl₄(Me₂SO)(Im)] in sterile and pyrogen-free physiological saline solution;
Group 5: RUT-GLU: 35 mg/kg body weight/die of Ruthenium (III) [trans-RuCl₄(Me₂SO)(Im)] and 23.5 mg/kg body weight/die of glutathione in sterile and pyrogen-free saline physiological solution;
Group 6: RUT-CYST: 35 mg/kg of body weight/die of Ruthenium (III) complex (lmH)[trans-RuCl₄(Me₂SO)(lm)] and 12 mg/kg body weight/die of cysteine in sterile and pyrogen-free saline physiological solution.

The reducing agents glutathione and cysteine were used in an equivalent ratio of 1 with the Ruthenium (III) complex. The redox reaction was immediate.

The administration took place after the analytical control of total reduction of the Ruthenium (III) to Ruthenium (II) complexes.

On day 16 from tumour implantation, the growth of the primary tumour as described in the previous example was evaluated.

On day 23 from tumour implantation, the mice were sacrificed and the metastases were evaluated as in previous example.

The experimental data obtained were processed by appropriate statistical tests and reported in Table 2, wherein the number and weight of lung metastases obtained in the treated group of animals are also reported.

**Table 2**

| Treatment | Metastases | | |
|---|---|---|---|
| | Number | Weight (mg) | %T/C |
| 1 Controls | 44.2±6.6 | 184.5±26.9 | 100 |
| 2 GLU | 47.0±3.9 | 138.8±20.5 | 75 |
| 3 CIST | 30.5±7.2 | 141.2±15.1 | 76 |
| 4 RUT | 27.5±3.0 | 83.6±4.5 | 45 |
| 5 RUT+GLU | 23.1±1.8 | 47.1±4.6 | 26 |
| 6 RUT + CYST | 19.2±3.4 | 44.2±7.5 | 24 |
| %T/C= percentage of the metastases weight in the treated animals/metastases weight in the controls. | | | |

From the data shown in Table 2, the antimetastatic effect of the Ruthenium (II) complexes according to the invention (groups 5 and 6) is clearly seen in respect to the effect of the corresponding compound of Ruthenium (III) (group 4); this effect refers both to the reduction of the number of metastases and, even more significantly, to the reduction of their weight.

### EXAMPLE 6: In vivo activity assays of the Ruthenium (III) and Ruthenium (II) complexes of the state of the art, in BD2F1 mice affected by Lewis lung carcinoma (LLC).

Groups of 7 BD2F1 female mice were inoculated by means of intramuscular injection on day 0 with cells of Lewis lung carcinoma (LLC). From day 8 to day 13 the animals were treated with the Ruthenium (III) complex [trans-RuCl₄(DMSO)(lm)](lmH), (compound of formula II) and with the Ruthenium (II) complex, trans-RuCl₂(DMSO)₄, both belonging to the state of the art. On day 20 from tumour implantation, the mice were sacrificed and the metastases evaluated. All the procedures of the experiments were carried out as reported in the previous examples.

The experimental data obtained, processed by appropriate statistical tests are shown in Table 3.

**Table 3**

| Compound (mg/kg/die) | Lung Metastases | | | |
|---|---|---|---|---|
| | Number | %T/C | Weight | %T/C |
| Controls | 24.4±4.6 | 100 | 191.3±35.7 | 100 |
| [trans-RuCl₄(DMSO)(lm)] (lmH) (35 mg/kg) | 9.4±1.5 | 38 | 47.6±11.5 | 25 |
| trans-RuCl₂(DMSO)₄ (70 mg/kg) | 19.6±1.9 | 82 | 85.5±19.3 | 45 |
| %T/C= percentage of the metastases weight or number in the treated animals/metastases weight or number in the controls. | | | | |

The data reported in Table 6, show that the Ruthenium (III) complex of formula II ([trans-RuCl₄(DMSO)(Im)] (lmH)) belonging to the state of the art is more effective in the reduction of lung metastases than the Ruthenium (II) complex (trans-RuCl₂(DMSO)₄) of the state of the art.

### Toxicity assays.

### EXAMPLE 7: Effects of the in vivo treatment of the compositions according to the present invention on mice affected by MCa mammary carcinoma.

Four groups, each of them composed of 7 CBA/Lac-kin female mice weighing 23±3 g on day 0 were inoculated with MCa mammary carcinoma cells according to the protocol described in the previous examples. From day 11 to day 16 after tumour implantation, the four groups of mice were treated intraperitoneally using a sterile insulin syringe, respectively with:
Group 1: Control: 10 ml/kg body weight/die of sterile and pyrogen-free physiological solution;
Group 2: CYST: 2 mg/kg body weight/die of cysteine in sterile and pyrogen-free physiological solution;
Group 3: RUT: 35 mg/kg body weight/die of Ruthenium (III) complex (lmH)[trans-RuCl₄(Me₂SO)(Im)] in sterile and pyrogen-free physiological solution;
Group 4: RUT-CYST: 35 mg/kg body weight/die of Ruthenium (III) complex , (ImH)[trans-RuCl₄(Me₂SO)(lm)] and 12 mg/kg body weight/die of cysteine in sterile and pyrogen-free physiological solution.

The reducing agent (cysteine) was used in a equivalent ratio of 1with the Ruthenium (III) complex. The redox reaction was immmediate.

The administration took place after analytical control of the complete reduction of the Ruthenium (III) to Ruthenium (II) complexes.

On day 17 from tumour implantation, the mice were sacrificed, the primary tumour and the spleen were removed and analysed as in the previous example. The results are reported in Table 4 which shows: the weight variation of the primary tumour (% variation with respect to the controls) from day 11 to day 17, the animal weight on day 11 and 17, the body weight variation (expressed in percentage with respect to the controls) and the spleen weight on day 17.

**Table 4**

| Treatment | % Variation of the primary tumour | Body weight (day 11) | Body Weight (day 17) | % variation | Spleen weight (day 17) |
|---|---|---|---|---|---|
| 1 Controls | - | 31.4±0.5 | 31.0±0.7 | - | 273± 13.3 |
| 2 CYST | - 6.4 | 30.5±0.6 | 28.8±0.6 | -2.5 | 297± 3.3 |
| 3 RUT | -30.3 | 30.0±1.0 | 26.5±0.8 | -8.8 | 197± 8.8 |
| 4 RUT+CYST | -13.6 | 30.8±0.6 | 29.0±0.4 | -2.3 | 282± 20.3 |

The data reported in the Table 4 indicate how the body weight and the spleen weight, which are indicators of the systemic toxicity of the treatment, turned out to be more similar to the control (group 1) when the mice were treated with the Ruthenium (II) complexes according to the invention (group 4) than when treated with Ruthenium (III) (group 3) only. These data are in agreement with a lower systemic toxicity of the Ruthenium (II) complexes according to the invention with respect to the Ruthenium (III) complexes of corresponding formula.

### EXAMPLE 8. Effects of the in vivo treatment of the compositions according to the present invention in mice affected by MCa mammary carcinoma.

Four groups each composed of 6 CBA/Lac kin female rats weighing 23±3 g. were inoculated on day 0 with tumour cells according to the protocol described in the previous examples. From day 12 to day 17, after tumour implantation, the four groups of mice were treated intraperitoneally using a sterile insulin syringe, respectively with:
Group 1: Control: 10 ml/kg body weight/die of sterile and pyrogen-free physiological solution;
Group 2: ASC: 6.69 mg/kg body weight/die of ascorbic acid in sterile and pyrogen-free physiological solution;
Group 3: RUT: 35 mg/kg body weight/die of Ruthenium (III) complex, (lmH)[trans-RuCl₄(Me₂SO)(Im)] in sterile and pyrogen-free physiological solution;
Group 4: RUT+ASC: 35 mg/kg body weight/die of Ruthenium (III) complex (lmH)[trans-RuCl₄(Me₂SO)(lm)] and 6.69 mg/kg body weight/ die of ascorbic acid in sterile and pyrogen-free physiological saline solution.

The reducing agent (ascorbic acid) was used in a equivalent ratio of 1 with the Ruthenium (III) complex. The redox reaction was immediate.

The administration took place after analytical control of the complete reduction of the Ruthenium (III) complex to Ruthenium (II).

On day 18 from tumour implantation, the primary tumour and the spleen were removed and weighed, as described previously.

The results are reported in Table 5 which shows: the weight variation of the primary tumour (variation in % with respect to the controls) from day 12 to day 18, the animal weight on days 12 and 18, and the body weight variation (variation in % with respect to the controls).

**Table 5**

| Treatment | (%) Variation primary tumour | Body weight (day 12) | Body weight (day 18) | (%) Variation |
|---|---|---|---|---|
| 1 Controls | - | 28.9±0.5 | 28.0±0.5 | - |
| 2 ASC | - 11 | 27.6±0.7 | 26.7±1.2 | - 0.1 |
| 3 RUT | - 33 | 27.0±0.9 | 23.3±0.8 | -10.8 |
| 4 RUT+ASC | - 28 | 25.4±1.0 | 25.1±0.9 | +2.1 |

The experiment confirms the lower toxicity of the Ruthenium (II) complexes of the invention (group 4) with respect to the prior art Ruthenium (III) complexes (group 3).

It is evident from the data reported in Tables 4 and 5 that the compositions according to the invention are less toxic than the corresponding Ruthenium (III) complexes of formula II. As a matter of fact, not only the decrease in body weight is lower, but also the spleen weight is substantially unaltered, contrary to what occurs in group 3 with the Ruthenium (III) compound.

As the result of all the activity and toxicity tests performed, it is therefore possible to conclude that the Ruthenium (II) compounds of the invention, are markedly more active than the corresponding Ruthenium (III) complexes and than the Ruthenium (II) complexes belonging to the state of the art. Moreover, unexpectedly, the complexes according to the invention are endowed with a lower systemic toxicity contrarily to what expected from the state of the art.

Altogether the results show that a new therapeutical alternative for the treatment of tumours with a higher and more selective efficacy and with lower systemic toxicity with respect to all known treatments based on ruthenium complexes is herein disclosed.

## Claims

1. A Ruthenium (II) complex of formula I: 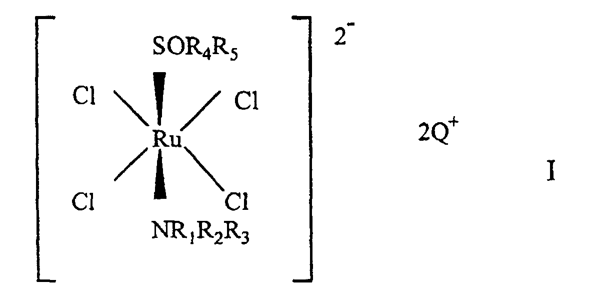 wherein,
**R**_{**1**}, **R**_{**2**}, **R**_{**3**}, either the same or different from one another, are chosen in the groups consisting of: H, C₁-C₆ alkyl either linear or branched, saturated or unsaturated, C₃-C₇ cycloalkyl, aryl; or NR₁R₂R₃ is a 5-7 membered nitrogen heterocycle, saturated or unsaturated, containing possibly 1 or more atoms selected in the group consisting O, S and N or N substituted with a C₁-C₄ alkylthio residue, aryl or benzyl; said nitrogen heterocycle being possibly benzocondensed and/or substituted with C₁-C₄ alkyl, C₁-C₄ alkoxy, C₁-C₄ alkylthio, aryl or benzyl;
and wherein:
**Q**^{**+**}= NH⁺R₁R₂R₃, wherein R₁, R₂ and R₃ maintain the above mentioned meanings;
and wherein:
**R**_{**4**} **and R**_{**5**}, either the same or different from one another, are selected in the group consisting of: H, C₁-C₆ alkyl, C₃-C₇ cycloalkyl, aryl; or R₄ and R₅ form together with the S atom to which they are bound, a 4-7-membered heterocycle,
for use in therapy.

2. The complex according to claim 1, wherein R₁, R₂ and R₃ are selected in the group consisting of: H, ethyl.

3. The complex according to claim 1, wherein NR₁R₂R₃ is a 5-membered nitrogen heterocycle selected in the group consisting of: imidazole, N-methylimidazole, pyrazole and oxazole.

4. The complex according to claim 1, wherein NR₁R₂R₃ is a 6-membered heterocycle selected in the group consisting of: pyridine, 3,5-lutidine and 4-methylpyridine.

5. The complex according to claim 1, wherein NR₁R₂R₃ is a 7-membered heterocycle selected in the group consisting of: azepine, diazepine and oxazepine.

6. The complex according to claim 1, wherein NR₁R₂R₃ is a benzocondensed nitrogen heterocycle selected in the group consisting of: indazole, isoquinoline, benzimidazole and 1,5,6-trimethyl-benzimidazole.

7. The complex according to anyone of claims 1-6, wherein R₄-SO-R₅ is selected in the group consisting of: dimethylsulfoxide and diethylsulfoxide.

8. The complex according to any of claims 1, 3 and 7, wherein NR₁R₂R₃ is imidazole, Q⁺ is imidazolium and R₄-SO-R₅ is dimethylsulfoxide.

9. The complex according to any of claims 1, 2 and 7, wherein NR₁R₂R₃ is imidazole, Q⁺ is ammonium and R₄-SO-R₅ is dimethylsulfoxide.

10. Pharmaceutical compositions containing the Ruthenium (II) complex as the active compound, as defined in anyone of claims 1-9, in combination with suitable excipients and/or diluents and/or pharmacologically acceptable stabilizers.

11. The pharmaceutical composition according to claim 10, **characterised in that** it is in the form of solution or suspension.

12. The pharmaceutical composition according to claim 10, **characterised** to be in the form of: gel cream, granular powder, tablet, pill, capsule or insert.

13. The pharmaceutical composition according to claim 10, wherein said Ruthenium (II) complexes are in combination with one or more antitumour drugs.

14. The pharmaceutical composition according to anyone of claims 10-13, preparable immediately before use by mixing the corresponding Ruthenium (III) complex with a physiologically compatible reducing agent.

15. The pharmaceutical composition according to claim 14, wherein the reducing agent is selected in the group consisting of: ascorbic acid, cysteine and glutathione.

16. The pharmaceutical composition according to any of claims 14 and 15, wherein the Ruthenium (III) complex is used in a ratio of equivalents with the reducing agent ranging from 5:1 to 1:2.

17. The pharmaceutical composition according to Claim 16, wherein the ratio of equivalents between the Ruthenium (III) complex and the reducing agent is 1.

18. A kit for the preparation of the Ruthenium (II) complex as defined in anyone of claims 1-9, wherein the first component is the corresponding Ruthenium (III) complex and the second is a reducing agent.

19. The kit according to claim 18, wherein both components are present in a solid form in separate containers to be mixed and solubilised immediately before use in a suitable solvent.

20. The kit according to claim 18, wherein both components are present in the form of solutions to be mixed immediately before use.

21. The kit according to claim 18, wherein the reducing agent is selected in the group consisting of: ascorbic acid, glutathione and cysteine.

22. The kit according to anyone of claims 18-21, wherein both components are present in a ratio of equivalents of the Ruthenium (III) complex with the reducing agent ranging from 5:1 to 1:2.

23. The kit according to claim 22, wherein the ratio of equivalents is 1.

24. Use of a Ruthenium (II) complex as defined in anyone of claims 1-9, for the preparation of medicaments for the prevention and treatment of tumours and metastases.

25. The use according to claim 24, wherein said tumours are solid and **characterised by** a high metastasising ability.

26. The use according to claim 25, wherein said tumours are selected in the group consisting of: colon carcinomas, mammary carcinomas, lung tumours and lung metastases from metastatic tumours.

27. The use according to anyone of claims 24-26, wherein the administration is parenteral, oral, topical or transdermal.

28. The use according to anyone of claims 24-27, wherein the Ruthenium (II) complex is prepared immediately before use, by mixing the corresponding Ruthenium (III) complex with a reducing agent.

29. The use according to claim 28, wherein the reducing agent is selected in the group consisting of: ascorbic acid, cysteine and glutathione.

## Patentansprüche

1. Ruthenium(II)-Komplex der Formel (I) zur therapeutischen Verwendung: 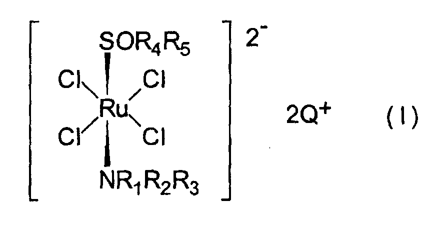 worin R₁, R₂ und R₃ identisch oder voneinander unterschiedlich sind und ausgewählt sind aus H, linearem oder verzweigtem, gesättigtem oder ungesättigtem C₁₋₆-Alkyl, C₃₋₇-Cycloalkyl und Aryl; oder NR₁R₂R₃ ist ein 5- bis 7-gliedriger, gesättigter oder ungesättigter Stickstoffheterocyclus, der gegebenenfalls 1 oder mehr Atome, ausgewählt aus O, S und N, oder mit einem C₁₋₄-Alkylthiorest, Aryl oder Benzyl substituiertem N, enthält; der Stickstoffheterocyclus kann gegebenenfalls Benzokondensiert und/oder mit C₁₋₄-Alkyl, C₁₋₄-Alkoxy, C₁₋₄-Alkylthio, Aryl oder Benzyl substituiert sein;
und worin:
Q⁺ = NH⁺R₁R₂R₃, worin R₁, R₂ und R₃ die oben angegebenen Bedeutungen beibehalten;
und worin:
R₄ und R₅ identisch oder voneinander verschieden sind und ausgewählt sind aus H, C₁₋₆-Alkyl,
C₃₋₇-Cycloalkyl und Aryl, oder R₄ und R₅ bilden zusammen mit dem S-Atom, an das sie gebunden sind, einen 4- bis 7-gliedrigen Heterocyclus.

2. Komplex gemäss Anspruch 1, worin R₁, R₂ und R₃ ausgewählt sind aus H und Ethyl.

3. Komplex gemäss Anspruch 1, worin NR₁R₂R₃ ein 5-gliedriger Heterocyclus ist, ausgewählt aus Imidazol, N-Methylimidazol, Pyrazol und Oxazol.

4. Komplex gemäss Anspruch 1, worin NR₁R₂R₃ ein 6-gliedriger Heterocyclus ist, ausgewählt aus Pyridin, 3,5-Lutidin und 4-Methylpyridin.

5. Komplex gemäss Anspruch 1, worin NR₁R₂R₃ ein 7-gliedriger Heterocyclus ist, ausgewählt aus Azepin, Diazepin und Oxazepin.

6. Komplex gemäss Anspruch 1, worin NR₁R₂R₃ ein Benzokondensierter Stickstoffheterocyclus ist, ausgewählt aus Indazol, Isochinolin, Benzimidazol und 1,5,6-Trimethyl-benzimidazol.

7. Komplex gemäss mindestens einem der Ansprüche 1 bis 6, worin R₄-SO-R₅ ausgewählt ist aus Dimethylsulfoxid und Diethylsulfoxid.

8. Komplex gemäss mindestens einem der Ansprüche 1, 3 und 7, worin NR₁R₂R₃ Imidazol ist, Q⁺ ist Imidazolium und R₄-SO-R₅ ist Dimethylsulfoxid.

9. Komplex gemäss mindestens einem der Ansprüche 1, 2 und 7, worin NR₁R₂R₃ Imidazol ist, Q⁺ ist Ammonium und R₄-SO-R₅ ist Dimethylsulfoxid.

10. Pharmazeutische Zusammensetzung, die den Ruthenium(II)-Komplex wie in mindestens einem der Ansprüche 1 bis 9 definiert, als aktive Verbindung in Kombination mit geeigneten Trägerstoffen und/oder Verdünnungsmitteln und/oder pharmakologisch annehmbaren Stabilisatoren enthält.

11. Pharmazeutische Zusammensetzung gemäss Anspruch 10, **dadurch gekennzeichnet, dass** sie in Form einer Lösung oder Suspension vorliegt.

12. Pharmazeutische Zusammensetzung gemäss Anspruch 10, **dadurch gekennzeichnet, dass** sie in Form einer Gelsalbe, eines granularen Pulvers, einer Tablette, einer Pille, einer Kapsel oder einer Einlage vorliegt.

13. Pharmazeutische Zusammensetzung gemäss Anspruch 10, worin die Ruthenium(II)-Komplexe mit einem oder mehreren Antitumorwirkstoffen kombiniert sind.

14. Pharmazeutische Zusammensetzung gemäss mindestens einem der Ansprüche 10 bis 13, herstellbar unmittelbar vor der Anwendung durch Vermischen des entsprechenden Ruthenium(III)-Komplexes mit einem physiologisch kompatiblen Reduktionsmittel.

15. Pharmazeutische Zusammensetzung gemäss Anspruch 14, worin das Reduktionsmittel ausgewählt ist aus Ascorbinsäure, Cystein und Glutathion.

16. Pharmazeutische Zusammensetzung gemäss mindestens einem der Ansprüche 14 und 15, worin der Ruthenium(III)-Komplex in einem Äquivalenzverhältnis zum Reduktionsmittel im Bereich von 5:1-1:2 verwendet wird.

17. Pharmazeutische Zusammensetzung gemäss Anspruch 16, worin das Äquivalenzverhältnis zwischen Ruthenium (III)-Komplex und Reduktionsmittel 1 beträgt.

18. Kit zur Herstellung des Ruthenium(II)-Komplexes wie in mindestens einem der Ansprüche 1 bis 9 definiert, worin die erste Komponente der entsprechende Ruthenium(III)-Komplex ist, und die zweite ist ein Reduktionsmittel.

19. Kit gemäss Anspruch 18, worin beide Komponenten in fester Form in getrennten Behältern vorliegen und unmittelbar vor der Verwendung in einem geeigneten Lösungsmittel vermischt und solubilisiert werden.

20. Kit gemäss Anspruch 18, worin beide Komponenten in Form von Lösungen vorliegen, die unmittelbar vor der Verwendung miteinander vermischt werden.

21. Kit gemäss Anspruch 18, worin das Reduktionsmittel ausgewählt ist aus Ascorbinsäure, Glutathion und Cystein.

22. Kit gemäss mindestens einem der Ansprüche 18 bis 21, worin beide Komponenten in einem Äquivalenzverhältnis von Ruthenium(III)-Komplex zu Reduktionsmittel im Bereich von 5:1-1:2 vorliegen.

23. Kit gemäss Anspruch 22, worin das Äquivalenzverhältnis 1 ist.

24. Verwendung eines Ruthenium(II)-Komplexes wie in mindestens einem der Ansprüche 1 bis 9 definiert, zur Herstellung eines Medikaments zur Vorbeugung und Behandlung von Tumoren und Metastasen.

25. Verwendung gemäss Anspruch 24, worin die Tumore fest und durch eine hohe Metastasenbildungsfähigkeit **gekennzeichnet** sind.

26. Verwendung gemäss Anspruch 25, worin die Tumore ausgewählt sind aus Colonkarzinomen, Mammakarzinomen, Lungentumoren und Lungenmetastasen aus metastatischen Tumoren.

27. Verwendung gemäss mindestens einem der Ansprüche 24 bis 26, worin die Verabreichung parenteral, oral, topisch oder transdermal erfolgt.

28. Verwendung gemäss mindestens einem der Ansprüche 24 bis 27, worin der Ruthenium(II)-Komplex unmittelbar vor der Verwendung durch Vermischen des entsprechenden Ruthenium(III)-Komplexes mit einem Reduktionsmittel hergestellt wird.

29. Verwendung gemäss Anspruch 28, worin das Reduktionsmittel ausgewählt ist aus Ascorbinsäure, Cystein und Glutathion.

## Revendications

1. Complexe de ruthénium (II) de formule I : 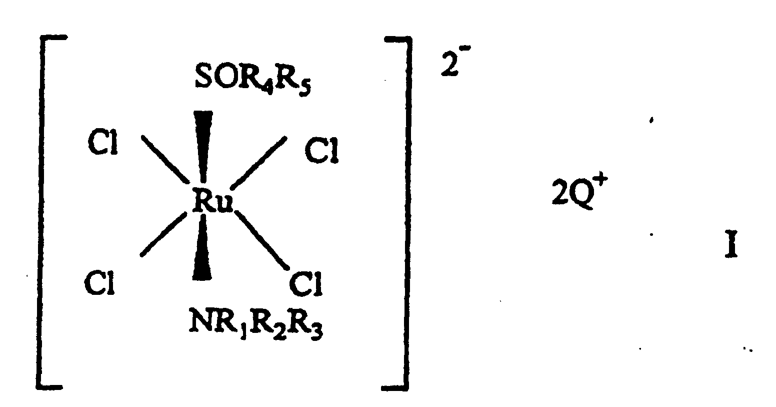 dans laquelle
R₁, R₂ et R₃, identiques ou différents les uns des autres, sont choisis dans le groupe constitué par : H, alkyle en C₁-C₆ linéaire ou ramifié, saturé ou insaturé, cycloalkyle en C₃-C₇, aryle ; ou NR₁R₂R₃ est un hétérocycle azoté à 5 - 7 chaînons, saturé ou insaturé, pouvant contenir 1 ou plusieurs atomes choisis dans le groupe constitué par O, S et N ou N substitué par un reste alkylthio en C₁-C₄, aryle ou benzyle ; ledit hétérocycle azoté pouvant être benzocondensé et/ou substitué par un groupe alkyle en C₁-C₄, alcoxy en C₁-C₄, alkylthio en C₁-C₄, aryle ou benzyle ;
et dans laquelle
Q⁺ = NH⁺R₁R₂R₃ dans laquelle R₁, R₂ et R₃ conservent les significations mentionnées ci-dessus ;
et dans laquelle
R₄ et R₅, identiques ou différents l'un de l'autre, sont choisis dans le groupe constitué par : H, alkyle en C₁-C₆, cycloalkyle en C₃-C₇, aryle ; ou R₄ et R₅ forment, avec l'atome S auquel ils sont liés, un hétérocycle à 4 - 7 chaînons,
pour l'utilisation en thérapie.

2. Le complexe selon la revendication 1, dans lequel R₁, R₂ et R₃, sont choisis dans le groupe constitué par : H, éthyle.

3. Le complexe selon la revendication 1, dans lequel NR₁R₂R₃ est un hétérocycle azoté à 5 - 7 chaînons choisi dans le groupe constitué par : un imidazole, un N-méthylimidazole, un pyrazole et un oxazole.

4. Le complexe selon la revendication 1, dans lequel NR₁R₂R₃ est un hétérocycle azoté à 6 chaînons choisi dans le groupe constitué par : une pyridine, une 3,5-lutidine, et une 4-méthylpyridine.

5. Le complexe selon la revendication 1, dans lequel NR₁R₂R₃ est un hétérocycle azoté à 7 chaînons choisi dans le groupe constitué par : une azépine, une diazépine et une oxazépine.

6. Le complexe selon la revendication 1, dans lequel NR₁R₂R₃ est un hétérocycle azoté benzocondensé choisi dans le groupe constitué par : un indazole, une isoquinoline, un benzimidazole et un 1,5,6-triméthylbenzimidazole.

7. Le complexe selon l'une quelconque des revendications 1-6, dans lequel R₄-SO-R₅ est choisi dans le groupe constitué par : un diméthylsulfoxyde et un diéthylsulfoxyde.

8. Le complexe selon l'une quelconque des revendications 1, 3 et 7, dans lequel NR₁R₂R₃ est un imidazole, Q⁺ est un imidazolium et R₄-SO-R₅ est un diméthylsulfoxyde.

9. Le complexe selon l'une quelconque des revendications 1, 2 et 7, dans lequel NR₁R₂R₃ est un imidazole, Q⁺ est un ammonium et R₄-SO-R₅ est un diméthylsulfoxyde.

10. Compositions pharmaceutiques contenant comme composé actif le complexe de ruthénium (II) défini dans l'une quelconque des revendications 1-9, en combinaison avec des excipients et/ou diluants et/ou stabilisants pharmaceutiquement acceptables.

11. La composition pharmaceutique selon la revendication 10, **caractérisée en ce qu'**elle est sous forme de solution ou de suspension.

12. La composition pharmaceutique selon la revendication 10, **caractérisée en ce qu'**elle est sous forme de gel, crème, poudre granulaire, comprimé, pilule, capsule ou insert.

13. La composition pharmaceutique selon la revendication 10, dans laquelle lesdits complexes de ruthénium (II) sont en combinaison avec un ou plusieurs médicaments antitumoraux.

14. La composition pharmaceutique selon l'une quelconque des revendications 10-13, préparable immédiatement avant usage par mélange du complexe de ruthénium (III) correspondant avec un agent réducteur physiologiquement compatible.

15. La composition pharmaceutique selon la revendication 14, dans laquelle l'agent réducteur est choisi dans le groupe constitué par : l'acide ascorbique, la cystéine et le glutathion.

16. La composition pharmaceutique selon l'une quelconque des revendications 14 et 15, dans laquelle le complexe de ruthénium (III) est utilisé dans un rapport d'équivalents pour l'agent réducteur allant de 5:1 à 1:2.

17. La composition pharmaceutique selon la revendication 16, dans laquelle le rapport des équivalents entre le complexe de ruthénium (III) et l'agent réducteur est 1.

18. Un ensemble pour la préparation de complexe de ruthénium (II) tel que défini dans l'une quelconque des revendications 1-9, dans lequel le premier composant est le complexe de ruthénium (III) correspondant et le second est un agent réducteur.

19. L'ensemble selon la revendication 18, dans lequel les deux composants sont présents sous forme solide en conteneurs séparés à mélanger et à solubiliser immédiatement avant usage dans un solvant approprié.

20. L'ensemble selon la revendication 18, dans lequel les deux composants sont présents sous forme de solutions à mélanger immédiatement avant usage.

21. L'ensemble selon la revendication 18, dans lequel l'agent réducteur est choisi dans le groupe constitué par : l'acide ascorbique, le glutathion et la cystéine.

22. L'ensemble selon l'une quelconque des revendications 18-21, dans lequel les deux composants sont présents dans un rapport d'équivalents de complexe de ruthénium (III) à l'agent réducteur allant de 5:1 à 1:2.

23. L'ensemble selon la revendication 22, dans lequel le rapport des équivalents est 1.

24. L'utilisation d'un complexe de ruthénium (II) tel que défini dans l'une quelconque des revendications 1-9, pour la préparation de médicaments pour la prévention et le traitement des tumeurs et des métastases.

25. L'utilisation selon la revendication 24, dans laquelle lesdites tumeurs sont solides et **caractérisées par** une forte capacité de métastase.

26. L'utilisation selon la revendication 25, dans laquelle lesdites tumeurs sont sélectionnées dans le groupe constitué par : les carcinomes du colon, les carcinomes mammaires, les tumeurs du poumon, et les métastases du poumon résultant de tumeurs métastatiques.

27. L'utilisation selon l'une quelconque des revendications 24-26, dans laquelle la voie d'administration est parentérale, orale, topique ou transdermique.

28. L'utilisation selon l'une quelconque des revendications 24-27, dans laquelle le complexe de ruthénium (II) est préparé immédiatement avant usage, en mélangeant le complexe de ruthénium (III) correspondant avec un agent réducteur.

29. L'utilisation selon la revendication 28, dans laquelle l'agent réducteur est choisi dans le groupe constitué par : l'acide ascorbique, la cystéine et le glutathion.
